# EUROPÄISCHE PATENTSCHRIFT

(11) **EP 0 694 031 B1**
(45) Veröffentlichungstag und Bekanntmachung des Hinweises auf die Patenterteilung: **15.09.1999**
(21) Anmeldenummer: 94913528.9
(22) Anmeldetag: 05.04.1994
(51) Int. Cl.: C07C 233/38, C07C 233/36, D06M 13/405, C11D 1/52

(54) **Verfahren zur Herstellung von Fettsäureamiden und ihre Verwendung**
Process for the preparation of fatty-acid amides and their utilisation
Procédé pour la préparation d'amides d'acides gras et leur utilisation

(30) Priorität: 13.04.1993 DE 4312008
(43) Veröffentlichungstag der Anmeldung: 31.01.1996
(73) Patentinhaber: Henkel Kommanditgesellschaft auf Aktien, 40191 Düsseldorf (DE)
(72) Erfinder: UPHUES, Günter, D-40789 Monheim (DE); PLOOG, Uwe, D-42781 Haan (DE); NEUMANN, Peter, D-40589 Düsseldorf (DE)
(86) Internationale Anmeldenummer: EP9401051
(87) Internationale Veröffentlichungsnummer: WO9424092

(56) Entgegenhaltungen:
- EP-A- 0 158 174
- EP-A- 0 379 923
- WO-A-92/18685
- WO-A-93/03130
- DE-A- 1 922 046
- DE-A- 2 911 198
- FR-A- 2 580 664
- US-A- 3 454 494

## Beschreibung

Die Erfindung betrifft ein Verfahren zur Herstellung von Fettsäureamiden mit verbesserter Kaltwasserdispergierbarkeit sowie die Verwendung dieser Fettsäureamide zur Herstellung von Textilbehandlungsmitteln.

### Stand der Technik

Für die Behandlung von Textilfasern, -garnen oder -geweben wird eine Vielzahl von Verbindungen oder Stoffgemischen vorgeschlagen, die den damit behandelten Textilien erwünschte Eigenschaften verleihen oder die Bestandteile von Mitteln zur Textilpflege sind. Je nach Art der angewendeten Wirkstoffe können dabei die Verarbeitungseigenschaften, die Trageeigenschaften der Textilien wie auch deren Pflege verbessert werden.

So beschreibt beispielsweise die amerikanische Patentschrift **US 23 40 881** wäßrige Dispersionen von Kondensationsprodukten, hergestellt aus einem Hydroxyalkylpolyamin und einem Fettsäureglycerid, die die Gleitfähigkeit und die Weichheit der damit behandelten Textilien verbessern.

Die **US 34 54 494** betrifft Fettsäurekondensationsprodukte aus Fettsäuren bzw. deren Estern mit mehrwertigen Aminen mit einem Zusatz an dispergierend wirkenden Polyglycolethern. Die Fettsäurekondensationsprodukte werden erhalten indem 2 Mol Fettsäure bzw. Ester mit 1 Mol der mehrwertigen Amine umgesetzt werden.

Die europäische Offenlegungsschrift EP-A1 0 379 923 beschreibt Textilbehandlungsmittel, herstellbar durch Umsetzung von oliphatischen C₈-₂₂ Monocarbonsäuren mit ggf. hydroxylsubstituierten Polyaminen und anschließende Neutralisation nicht eingesetzter Aminogruppen, wobei das Textilbehandlungsmittel zwingend Dispersionsbeschleuniger aus der Gruppe der Monosaccharide vom Typ der Aldosen und Ketosen und den hieraus durch Hydrierung abgeleiteten Polyhydroxyverbindungen, der Polyole wie insbesondere Pentaerythrit, Dipentaerythrit, Trimethylolpropan, der Alkylglucoside, der Sorbitanester, an die gewünschtenfalls Ethylenoxid angelagert ist, und der natürlichen und synthetischen hydrophilen Polymere enthält.

Die deutsche Patentschrift **DE-PS 19 22 046** beschreibt Waschmittel mit einem Gehalt an Fettsäurekondensationsprodukten, die von ihrer Herstellung her dispergierend wirkende Fettsäureglyceride enthalten; in der deutschen Patentschrift DE-**PS 19 22 047** werden diese Stoffe auch als Textilweichmacher für insbesondere flüssige Wäschenachbehandlungsmittel beschrieben. Diese und ähnliche Mittel lassen sich in Wasser dispergieren, indem man sie in heißes Wasser einbringt und meist hohe Scherkräfte anwendet, oder indem man das von der Herstellung her noch geschmolzene Kondensationsprodukt in Wasser dispergiert. Wegen des erforderlichen Aufwandes nimmt daher meist der Hersteller die Dispergierung vor und liefert dem Anwender die Dispersionen, was mit der Lagerung und dem Transport erheblicher Mengen Wasser verbunden und somit aus ökonomischer Sicht wenig rentabel ist.

Nach der Lehre der deutschen Patentanmeldungen **DE-A1 35 30 302, DE-A1 37 30 792, DE-A1 39 01 820** und **DE-A1 41 11 648** (Henkel) kann man die Dispergierbarkeit derartiger Stoffe durch den Zusatz geeigneter Dispersionsbeschleuniger, beispielsweise Glucose, Sorbit, Mannit, Pentaerythrit, Alkyl-polyglucoside, Sorbitanester, Gelatine sowie ungesättigte Fettalkohole und Fettsäuren verbessern. Am Markt etabliert sind ferner Produkte, die als Dispersionsbeschleuniger Mischungen hochethoxylierter Talgalkohole und 1,2-Propylenglycol enthalten.

Trotzdem wird in der Praxis immer wieder beobachtet, daß Chargen an Kondensationsprodukten von Fettsäuren und mehrwertigen Aminen anfallen, die sich in Wasser nicht oder nur unzureichend dispergieren lassen. Diese Unsicherheit im Hinblick auf die Produktqualität stellt einen erheblichen wirtschaftlichen Nachteil dar, der die Verwendbarkeit der genannten Stoffe als begehrte Textilbehandlungsmittel einschränkt. Ein weiteres Problem besteht ferner darin, daß selbst solche Produkte des Stands der Technik, die über eine zufriedenstellende Dispergierbarkeit verfügen, diese in der Regel irreversibel einbüßen, wenn sie an der Luft gelagert werden.

Die Aufgabe der Erfindung bestand somit darin, neue Fettsäureamide zur Verfügung zu stellen, die frei von den geschilderten Nachteilen sind.

### Beschreibung der Erfindung

Gegenstand der Erfindung ist ein Verfahren zur Herstellung von Fettsäureamiden mit verbesserter Kaltwasserdispergierbarkeit, bei dem man
a) Fettsäuren der Formel **(I),**

   **R**^{**1**}**CO-OH** (I)

   in der R¹CO für einen aliphatischen Acylrest mit 8 bis 22 Kohlenstoffatomen und 0 oder 1 bis 3 Doppelbindungen steht, oder deren Ester mit mehrwertigen Aminen der Formel **(II),** 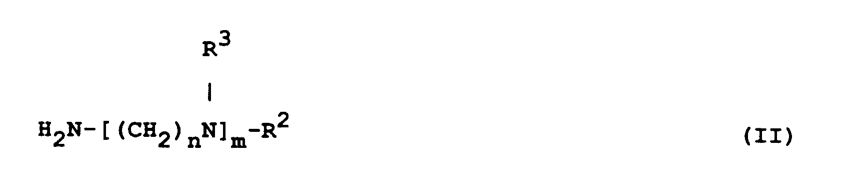 in der R² für Wasserstoff, eine Methyl-, Ethyl-Hydroxyethyl- oder eine -(CH₂)ₙ-NHR³-Gruppe, R³ für Wasserstoff, eine Methyl-, Ethyl- oder Hydroxy-ethylgruppe, n für Zahlen von 2 bis 4 und m für Zahlen von 1 bis 4 steht, kondensiert,
b) nichtumgesetzte Aminogruppen mit Säuren partiell neutralisiert und
c) Dispersionsbeschleuniger der Formel **(III)** zusetzt, 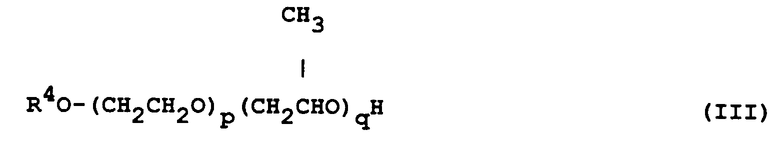 in der R⁴ für einen Alkyl- oder Alkenylrest mit 12 bis 22 Kohlenstoffatomen, p für 0 oder Zahlen von 1 bis 5 und q für Zahlen von 1 bis 5 steht,
**dadurch gekennzeichnet,** das die Fettsäuren bzw. deren Ester und die mehrwertigen Amine im molaren Verhältnis von 1,3 : 1 bis 1,7 : 1 eingesetzt werden.

Überraschenderweise wurde gefunden, daß der Zusatz der ausgewählten nichtionischen Tenside zu den Kondensationsprodukten nicht nur Produkten mit einer gegenüber dem nächstliegenden Stand der Technik deutlich verbesserten Dispergierbarkeit führt, sondern die Kondensate ihre vorteilhaften Eigenschaften auch bei längerer Lagerung an der Luft behalten.

### Ausgangsstoffe

Als Komponente I zur Herstellung der erfindungsgemäßen Fettsäureamide kommen **Fettsäuren** der Formel **(I)** in Betracht. Typische Beispiele sind Capronsäure, Caprinsäure, Caprylsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, wie sie beispielweise bei der Druckspaltung von Fetten und Ölen anfallen. Die Fettsäuren können auch in Form ihrer C₁₋₄-Alkylester, insbesondere ihrer Methylester, sowie ihrer Glycerinester, insbesondere in Form von Triglyceriden, eingesetzt werden.

Vorzugsweise werden technische Palm- oder Talgfettsäuren der Formel **(I)** eingesetzt, in der R¹CO für einen Acylrest mit 16 bis 18 Kohlenstoffatomen und 0 und/oder 1 Doppelbindung steht.
Die als Komponente II) in Betracht kommenden **mehrwertigen Amine** leiten sich vorzugsweise von gegebenfalls hydroxylsubstituiertem Ethylendiamin oder Diethylentriamin ab. Typische Beispiele sind Dihydroxyethylendiamin, Hydroxyethyldiethylen-diamin , Hydroxypropyldiethylentriamin und insbesondere Aminoethylethanolamin. Außerdem geeignet sind N,N-Dimethyl-1,3diaminopropan, Triethylentetramin oder Tetraethylenpentamin.

Im Hinblick auf die anwendungstechnischen Eigenschaften der resultierenden Kondensationsprodukte hat es sich als optimal erwiesen, mehrwertige Amine der Formel **(II)** einzusetzen, in der R² für eine Hydroxyethyl- oder (CH₂)₂NH₂-Gruppe, R³ für Wasserstoff, n für 2 und m für 1 oder 2 steht.

### Kondensationsreaktion

Bei der Herstellung der Kondensationsprodukte werden die Fettsäuren oder deren Ester und die mehrwertigen Amine in einem molaren Verhältnis von 1,3 :1 bis 1,7:1 eingesetzt. Man erhitzt die Komponenten über einen Zeitraum von 0,5 bis 5, vorzugsweise 1 bis 2 h auf eine Temperatur von 170 bis 210, vorzugsweise 180 bis 200°C bis die Fettsäure bzw. der Fettsäureester praktisch vollständig umgesetzt sind und läßt die Mischung anschließend weitere 5 bis 60, vorzugsweise 15 bis 30 min bei der Reaktionstemperatur nachreagieren. Wird die Herstellung besonders hellfarbiger Produkte angestrebt, empfiehlt es sich, die Kondensation unter Ausschluß von Luftsauerstoff und gegebenenfalls in Gegenwart eines Reduktionsmittels, z. B. unterphosphoriger Säure, durchzuführen.

Im Anschluß an die Kondensation neutralisiert man die nichtumgesetzten Aminogruppen mit niedermolekularen organischen Carbonsäuren, Hydroxycarbonsäuren oder anorganischen Säuren, wie z. B. Essigsäure, Milchsäure oder Phosphorsäure, indem man unter Salzbildung beispielsweise die Schmelze des Fettsäurekondensationsproduktes mit der berechneten Menge Säure vermischt oder das Aminsalz durch Auflösen oder Dispergieren des Umsetzungsproduktes in der organischen Säure oder einer Lösung der organischen Säure in Wasser bildet. Die zur Salzbildung verwendete Säure kann erfindungsgemäß in solchen Mengen eingesetzt werden, wie sie zur 20 bis 80, vorzugsweise 30 bis 60 Mol.-%igen Neutralisation erforderlich ist.

### Dispersionsbeschleuniger

Als Dispersionsbeschleuniger kommen vergleichsweise hydrophobe, nichtionische Tenside in Betracht, die der Formel (III) folgen. Typische Beispiele sind Anlagerungsprodukte von durchschnittlich 1 Mol Ethylenoxid und 2 Mol Propylenoxid, 2 Mol Ethylenoxid und 4 Mol Propylenoxid, 5 Mol Ethylenoxid und 1 Mol Propylenoxid sowie 2 bis 3 Mol Propylenoxid an C_{12/18}-Kokosfettalkohol, C_{16/18}-Talgfettalkohol oder C_{16/18}-Palm-fettalkohol.

Im Hinblick auf die dispergierenden Eigenschaften der resultierenden Produkte haben sich Dispersionsbeschleuniger bewährt, die der Formel **(III)** folgen, in der R⁴ für einen Alkylrest mit 12 bis 18 Kohlenstoffatomen steht und bei denen das Verhältnis von n : m im Bereich von 1 : 2 bis 5 : 1 liegt.

Der Zeitpunkt, an dem die Dispersionsbeschleuniger den Kondensationsprodukten zugegeben werden, ist unkritisch. Vorzugsweise führt man die Kondensation in Gegenwart der Dispersionsbeschleuniger durch oder mischt sie nach Abschluß der Reaktion den Partialneutralisaten zu. Die Einsatzmenge kann 0,5 bis 15, vorzugsweise 1 bis 10 Gew.-% betragen. Falls gewünscht, können auch weitere bekannte Dispersionsbeschleuniger, wie beispielsweise Monosaccharide vom Typ der Aldosen und Ketosen, die hieraus durch Hydrierung abgeleiteten Polyhydroxyverbindungen, Pentaerythrit, Trimethylolpropan, Alkyl-glucoside, Sorbitanester, Polysorbate sowie hydrophile Polymere auf Basis von Kohlenhydraten oder ungesättigten Alkoholen oder Carbonsäuren, mitverwendet werden.

### Gewerbliche Anwendbarkeit

Die erfindungsgemäßen Fettsäureamide können beispielsweise in Form von Pulvern, Schuppen oder Pellets konfektioniert werden und lassen sich leicht in Wasser, insbesondere auch in kaltem Wasser, zu stabilen Dispersionen verarbeiten. Hierzu genügt das Vermischen mit Wasser und anschließendes leichtes Umrühren. Die erhaltenen Dispersionen sind außerordentlich stabil und neigen nicht zur Entmischung. Sie lassen sich im Textilbereich in vielfältiger Weise zur Behandlung von Fasern, Garnen oder Geweben nach dem Auszieh-, Tauchschleuder-, Foulard-oder Sprühverfahren einsetzen.

Ein weiterer Gegenstand der Erfindung betrifft daher Textilbehandlungsmittel, enthaltend 10 bis 100, vorzugsweise 50 bis 95 Gew.-% der erfindungsgemäßen Fettsäureamide.

Bei der Verwendung der erfindungsgemäßen Textilbehandlungsmittel in Waschmitteln bewirken diese eine verbesserte Reinigungswirkung und/oder eine Avivage der damit gewaschenen Wäsche. Die erfindungsgemäßen Textilbehandlungsmittel können schließlich auch als Bestandteile von Nachbehandlungsmitteln für gewaschene Textilien verwendet werden, wodurch die Textilien weich und antistatisch werden. Die Nachbehandlung der gewaschenen Textilien kann üblicherweise im letzten Spülbad aber auch während des Trocknens in einem automatischen Wäschetrockner erfolgen, wobei man entweder die Wäsche während des Trocknens mit einer Dispersion des Mittels besprüht oder indem man ein flexibles, textiles Flächengebilde benutzt, das dem Substrat als Träger dient.

Schließlich betrift ein weiterer Gegenstand der Erfindung die Verwendung der erfindungsgemäßen Fettsäureamide zur Herstellung von Textilbehandlungsmitteln zur Veredlung von Fasern, Garnen oder Geweben sowie beim Waschen von Textilien sowie zur Nachbehandlung gewaschener Wäsche, in denen sie in Mengen von 10 bis 100, vorzugsweise 50 bis 95 Gew.-% - bezogen auf den Feststoffgehalt der Mittel enthalten sein können.

Die folgenden Beispiele sollen den Gegenstand der Erfindung näher erläutern, ohne ihn darauf einzuschränken.

### Beispiele

### I. Herstellbeispiele

### Beispiel 1:

In einem 4-1-Vierhalskolben mit Rührer, Thermometer, Gasüberleitungsrohr und Destillationsaufsatz wurden 2410 g (8,9 mol) einer technischen Stearinsäure unter Überleiten von Stickstoff geschmolzen. Bei 80°C wurde ein Gemisch aus 200,5 g (1,95 mol) Diethylentriamin und 284,5 g (1,95 mol) Triethylentetramin zugesetzt. Innerhalb von 2 h wurde die Temperatur auf 200°C gesteigert und die Mischung weitere 1,5 h hierbei unter Rühren belassen. Anschließend wurde auf 120°C abgekühlt. Im Verlauf der Reaktion war eine Destillatmenge von 177 g angefallen. Die Säurezahl des Produktes betrug 4,8, der Gehalt an titrierbarem Stickstoff 2,25 Gew.-%. Nach Erreichen der 120°C wurde das Produkt unter Zugabe von 212,3 g Essigsäure partiell neutralisiert, wobei 2908 g eines spröden Feststoffes anfielen.

### Beispiel 2:

560 g des gemäß Beispiel 1) erhaltenen Produktes wurden bei 120°C mit 60 g eines Adduktes von 2 Mol Ethylenoxid und 4 Mol Propylenoxid an einen C_{12/18}-Kokosfettalkohols (Dehypon^{(R)} LT 24, Henkel KGaA, Düsseldorf/FRG) versetzt. Anschließend wurde das Produkt auf einer Kühlwalze geschuppt. Die Trübungstitrationszahl (TTZ) des Dehypon^{(R)} LT24 gemäß DIN 53 989 betrug 17,8.

### Beispiel 3:

Beispiel 2) wurde unter Einsatz von 60 g eines Adduktes von 2 Mol Propylenoxid an einen technischen Cetyl/Oleylalkohol (Dehypon^{(R)} OCP 502, Henkel KGaA, Düsseldorf/FRG) wiederholt. Die TTZ des Dehypon^{(R)} OCP 502 betrug 11,5.

### Veraleichsbeispiel V1:

Beispiel 2) wurde unter Einsatz von 60 g eines Adduktes von 40 mol Ethylenoxid an einen C_{16/18}-Talgfettalkohol sowie 33,4 g 1,2-Propylenglycol wiederholt.

### Beispiel 4:

Analog Beispiel 1) wurden 378 g (1,4 mol) einer technischen Stearinsäure geschmolzen und bei 80°C mit 97,5 g (0,94 mol) Aminoethylethanolamin versetzt. Innerhalb von 1,5 h wurde die Mischung bis auf 180°C erhitzt und solange bei dieser Temperatur gerührt, bis die Säurezahl bis auf einen Wert von 5,5 abgesunken war. Im Verlauf der Reaktion wurden 26,4 g Destillat abgenommen. Die auf 110°C abgekühlte Schmelze wurde mit 20,5 g Milchsäure partiell neutralisiert (Neutralisationsgrad : ca. 80 %) und mit 30 g eines Adduktes von 2 Mol Propylenoxid an einen technischschen Cetyl/Oleylalkohol (Dehypon^{(R)} OCP 502, Henkel KGaA, Düsseldorf/FRG) vermischt. Anschließend wurde das Produkt auf einer Kühlwalze geschuppt.

### II. Überprüfung der Dispergierbarkeit

### a) Bewertung der Feinteiliakeit

7,5 g der geschuppten Produkte wurden in einer 125-ml-Weithals-Schraubdeckelflasche mit 92,5 g Wasser (30°C, 14°d) übergossen. Die Flasche wurde verschlossen, kurz umgeschwenkt und 16 h gelagert. Nach nochmaligem Umschwenken wurde über eine Nutsche, die mit einem feinmaschigen, schwarz eingefärbten Polyamidgewebe belegt war, bei 150 mbar abgesaugt. Nach dem Trocknen des Gewebes wurde die Feinteiligkeit des Rückstandes auf einer Skala von 1 (feinst dispers) bis 6 (grob dispers, viele große Klümpchen) visuell beurteilt. Die Ergebnisse sind in Tab.1 zusammengefaßt.

### b) Bewertung nach Laaeruna an der Luft

10 g der geschuppten Produkte wurden in einer Porzellanschale mit 10 cm Durchmesser 5 d an der Luft (30-40 % rel. Feuchtigkeit) gelagert und dann wie unter a) angegeben beurteilt. Die Ergebnisse sind in Tab.1 zusammengefaßt.

**Tab.1:**

| Beurteilung der Feinteiligkeit | | |
|---|---|---|
| Bsp. | Feinteiligkeit | |
| | nach 16 h | nach 5 d |
| 2 | 2 | 3 |
| 3 | 2 | 2 |
| 4 | 2 | 2 |
| V1 | 4,5 | 6 |

## Patentansprüche

1. Verfahren zur Herstellung von Fettsäureamiden mit verbesserter Kaltwasserdispergierbarkeit, bei dem man
a) Fettsäuren der Formel **(I),**
**R**^{**1**}**CO-OH** (I)
in der R¹CO für einen aliphatischen Acylrest mit 8 bis 22 Kohlenstoffatomen und 0 oder 1 bis 3 Doppelbindungen steht, oder deren Ester mit mehrwertigen Aminen der Formel **(II)**, 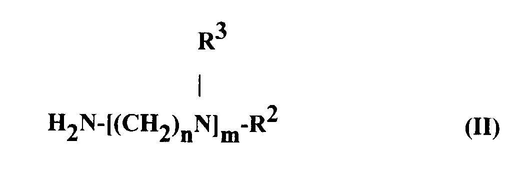 in der R² für Wasserstoff, eine Methyl-, Ethyl- Hydroxyethyl- oder eine-(CH₂)ₙ-NHR³-Gruppe, R³ für Wasserstoff, eine Methyl-, Ethyl- oder Hydroxyethylgruppe, n für Zahlen von 2 bis 4 und m für Zahlen von 1 bis 4 steht, kondensiert,
b) nichtumgesetzte Aminogruppen mit Säuren partiell neutralisiert und
c) Dispersionsbeschleuniger der Formel **(III)** zusetzt, 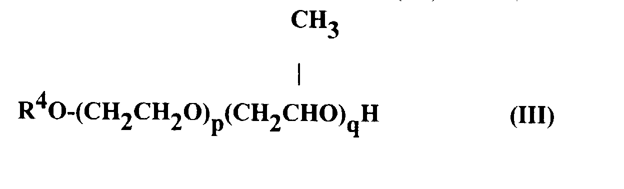 in der R⁴ für einen Alkyl- oder Alkenylrest mit 12 bis 22 Kohlenstoffatomen, p für 0 oder Zahlen von 1 bis 5 und q für Zahlen von 1 bis 5 steht,
**dadurch gekennzeichnet,** das die Fettsäuren bzw. deren Ester und die mehrwertigen Amine im molaren Verhältnis von 1,3 : 1 bis 1,7 : 1 eingesetzt werden.

2. Verfahren nach Anspruch 1, **dadurch gekennzeichnet**, daß man Fettsäuren der Formel (I) einsetzt, in der R¹CO für einen Acylrest mit 16 bis 18 Kohlenstoffatomen und 0 oder 1 Doppelbindung steht.

3. Verfahren nach den Ansprüchen 1 und 2, **dadurch gekennzeichnet**, daß man mehrwertige Amine der Formel (II) einsetzt, in der R² für eine Hydroxyethyl- oder (CH₂)₂NH₂-Gruppe, R³ für Wasserstoff, n für 2 und m für 1 oder 2 steht

4. Verfahren nach den Ansprüchen 1 bis 3, **dadurch gekennzeichnet,** daß man die Kondensation über einen Zeitraum von 0,5 bis 5 h bei einer Temperatur von 170 bis 210 °C durchführt.

5. Verfahren nach den Ansprüchen 1 bis 4, **dadurch gekennzeichnet,** daß man die nichtumgesetzten Aminogruppen zu 20 bis 80 Mol-% neutralisiert.

6. Verfahren nach den Ansprüchen 1 bis 5, **dadurch gekennzeichnet,** daß man die nichtumgesetzten Aminogruppen mit Essigsäure, Milchsäure und/oder Phosphorsäure neutralisiert.

7. Verfahren nach den Ansprüchen 1 bis 6, **dadurch gekennzeichnet**, daß man die Dispersionsbeschleuniger in einer Menge von 0,5 bis 15 Gew.-% zusetzt.

8. Verwendung von Fettsäureamiden nach Anspruch 1 zur Herstellung von Textilbehandlungsmitteln, die frei sind von Dispersionsbeschleunigern aus der Gruppe der Monosaccharide vom Typ der Aldosen und Ketosen und den hieraus durch Hydrierung abgeleiteten Polyhydroxyverbindungen, der Polyole wie insbesondere Pentaerythrit, Dipentaerythrit, Trimethylolpropan, der Alkylglucoside, der Sorbitanester, an die gewünschtenfalls Ethylenoxid angelagert ist, und natürlichen und synthetischen hydrophilen Polymeren.

## Claims

1. A process for the production of fatty acid amidos with improved dispersibility in cold water, in which
a) fatty acids corresponding to formula (I):
R¹CO-OH (I)
in which R¹CO is an aliphatic acyl radical containing 8 to 22 carbon atoms and 0 or 1 to 3 double bonds,
or esters thereof are condensed with polyfunctional amines corresponding to formula (II): 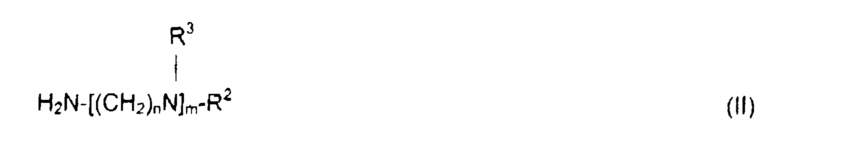 in which R² is hydrogen, a methyl, ethyl, hydroxyethyl or -(CH₂)ₙ-NHR³ group, R³ is hydrogen, a methyl, ethyl or hydroxyethyl group, n is a number of 2 to 4 and m is a number of 1 to 4,
b) unreacted amino groups are partly neutralized with acids and
c) dispersion accelerators corresponding to formula (III): 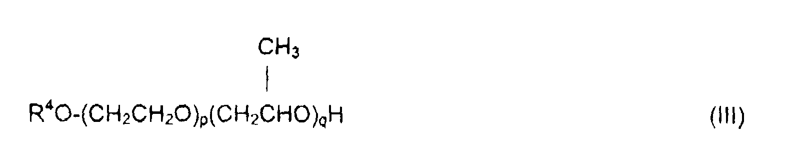 in which R⁴ is an alkyl and/or alkenyl radical containing 12 to 22 carbon atoms, p = 0 or is a number of 1 to 5 and q is a number of 1 to 5,
are added,
characterized in that the fatty acids or their esters and the polyfunctional amines are used in a molar ratio of 1.3:1 to 1.7:1

2. A process as claimed in claim 1, characterized in that fatty acids corresponding to formula (I), in which R¹CO is an acyl radical containing 16 to 18 carbon atoms and 0 or 1 double bond, are used.

3. A process as claimed in claims 2 and 3, characterized in that polyfunctional amines corresponding to formula (II), in which R² is a hydroxyethyl or (CH₂)₂NH₂ group, R³ is hydrogen, n is 2 and m is 1 or 2, are used.

4. A process as claimed in claims 1 to 3, characterized in that the condensation is carried out for 0.5 to 5 h at a temperature of 170 to 210°C.

5. A process as claimed in claims 1 to 4, characterized in that 20 to 80 mole-% of the unreacted amino groups are neutralized.

6. A process as claimed in claims 1 to 5, characterized in that the unreacted amino groups are neutralized with acetic acid, lactic acid and/or phosphoric acid.

7. A process as claimed in claims 1 to 6, characterized in that the dispersion accelerators are added in a quantity of 0.5 to 15% by weight.

8. The use of fatty acid amides as claimed in claim 1 for the production of textile treatment compositions which are free from dispersion accelerators from the group of monosaccharides of the aldose and ketose type and the polyhydroxy compounds derived therefrom by hydrogenation, polyols such as, in particular, pentaerythritol, dipentaerythritol or trimethylol propane, alkyl glucosides, sorbitan esters, onto which ethylene oxide is optionally added, and natural and synthetic hydrophilic polymers.

## Revendications

1. Procédé de préparation d'amides d'acides gras ayant une capacité améliorée de dispersion dans l'eau froide, dans lequel ;
a) on condense des acides gras de formule (I)
R¹CO-OH (I)
dans laquelle R¹CO représente un reste acyle aliphatique ayant de 8 à 22 atomes de carbone et 0 ou 1 à 3 doubles liaisons ou leurs esters, avec des amines polyfonctionnelles de formule (II) 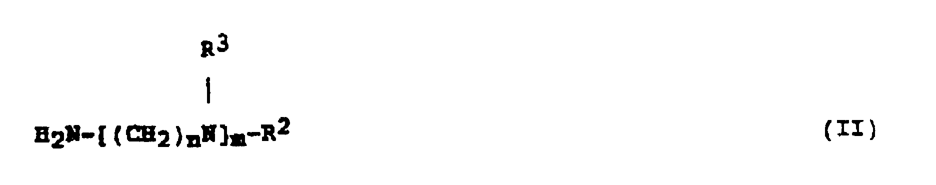 dans laquelle R² représente de l'hydrogène, un groupe méthyle, éthyle, hydroxyéthyle ou un groupe (CH₂)ₙ-NHR³, (R³ représente de l'hydrogène , un groupe méthyle, éthyle, ou hydroxyéthyle, n représente un nombre allant de 2 à 4 et m représente un nombre allant de 1 à 4,
b) on neutralise partiellement les groupes amino qui n'ont pas réagi avec des acides,
c) on ajoute des accélérateurs de dispersion de formule (III) 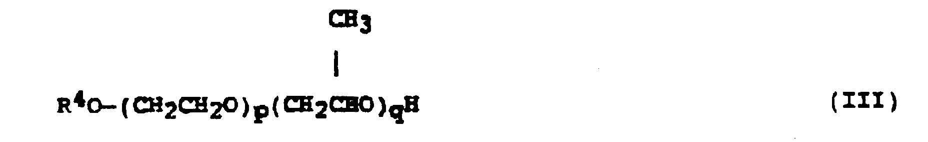 dans laquelle R⁴ représente un radical alkyle ou alkényle ayant de 12 à 22 atomes de carbone, p représente 0 ou un nombre allant de 1 à 5 et q représente un nombre allant de 1 à 5,
caractérisé en ce que
les acides gras ou leurs esters et les amimes polyfonctionnelles sont mis en oeuvre dans un rapport molaire de 1,3 : 1 à 1,7 : 1.

2. Procédé selon la revendication
caractérisé en ce qu'
on met en oeuvre des acides gras de formule (I) dans laquelle R¹CO représente un reste acyle ayant de 16 à 18 atomes de carbone et 0 ou 1 double liaison.

3. Procédé selon les revendications 1 et 2,
caractérisé en ce qu'
on met en oeuvre des amines polyfonctionnelles de formule (II) dans laquelle R² représente un groupe hydroxyéthyle où (CH₂)₂NH₂, n représente 2 et m représente 1 ou 2, et R³ représente de l'hydrogène.

4. Procédé selon les revendications 1 à 3,
caractérisé en ce qu'
on effectue la condensation pendant un laps de temps de 0,5 à 5 h à une température de 170 à 210°C.

5. Procédé selon les revendications 1 à 4,
caractérisé en ce qu'
on neutralise les groupes amino qui n'ont pas réagi pour 20 à 80 % mol.

6. Procédé selon les revendications 1 à 5,
caractérisé en ce qu'
on neutralise les groupes amino qui n'ont pas réagi avec de l'acide acétique, de l'acide lactique et/ou de l'acide phosphorique.

7. Procédé selon les revendications 1 à 6,
caractérisé en ce qu'
on ajoute l'accélérateur de dispersion en une quantité allant de 0,5 à 15 % en poids.

8. Utilisation d'amides d'acide gras selon la revendication 1, pour la production d'agents de traitement pour les textiles, qui sont dépourvus d'accélérateurs de dispersion choisis dans le groupe des monosaccharides, du type des aldoses, des ketoses, des composés polyhydroxylés qui en dérivent par hydrogénation, des polyols comme en particulier le pentaérythritol, le dipentaérychritol, le triméthylol propane, des alkylglycosides, des esters de sorbitanne, sur lesquels un oxyde d'éthylène est additionné si besoin, et des polymères hydrophiles naturels et synthétiques.
